(19) Europäisches Patentamt
European Patent Office
Office européen des brevets

(11) Numéro de publication: **0 030 198**
**B1**

(12) # FASCICULE DE BREVET EUROPEEN

(45) Date de publication du fascicule du brevet: **02.03.83**

(21) Numéro de dépôt: **80401725.9**

(22) Date de dépôt: **02.12.80**

(51) Int. Cl.³: **C 07 D 513/04,**
C 07 D 519/00,
A 61 K 31/435,
A 61 K 31/44,
A 61 K 31/47,
A 61 K 31/495,
A 61 K 31/50,
A 61 K 31/505
//C07D217/02, C07D217/26,
C07D277/40, (C07D513/04,
277/00, 221/00),
(C07D519/00, 513/00,
495/00)

(54) **Nouveaux dérivés de l'isoquinoléine, leur préparation et les médicaments qui les contiennent.**

(30) Priorité: **04.12.79 FR 7929751**
**17.10.80 FR 8022260**

(43) Date de publication de la demande:
**10.06.81 Bulletin 81/23**

(45) Mention de la délivrance du brevet:
**02.03.83 Bulletin 83/9**

(84) Etats contractants désignés:
**AT BE CH DE FR GB IT LI LU NL SE**

(56) Documents cités:
**FR - A - 2 320 098**
**FR - A - 2 351 655**
**FR - A - 2 378 030**

(73) Titulaire: **RHONE-POULENC SANTE**
**Les Miroirs 18 Avenue d'Alsace**
**F-92400 Courbevoie Cedex (FR)**

(72) Inventeur: **Farge, Daniel**
**30, rue des Pins Sylvestres**
**F-94320 Thiais (FR)**
Inventeur: **Jossin, Alain**
**14, Parc de Béarn**
**F-92210 St-Cloud (FR)**
Inventeur: **Ponsinet, Gérard**
**16, rue Lachevardière**
**F-94370 Sucy-en-Brie (FR)**
Inventeur: **Reisdorf, Daniel**
**39, avenue de la République**
**F-94320 Thiais (FR)**

(74) Mandataire: **Gaumont, Robert et al,**
**RHONE-POULENC RECHERCHES Service Brevets**
**Pharma 25, Quai Paul Doumer**
**F-92408 Courbevoie Cedex (FR)**

**EP 0 030 198 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Nouveaux dérivés de l'isoquinoléine, leur préparation et les médicaments qui les contiennent

Dans BE - A - 844 927 ont été décrites les thiazolo [3,4-b] isoquinoléines de formule générale:

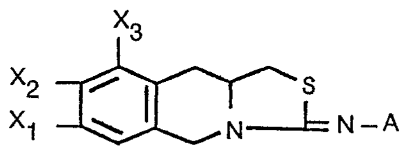

dans laquelle A représente pyridyl-3 ou -4 ou isoquinolyl-5 et $X_1$, $X_2$ et $X_3$ représentent notamment des atomes d'hydrogène.

Ces produits manifestent une activité analgésique et antipyrétique.

Dans BE - A - 863 083 ont été décrites les thiazolo [3,4-b] isoquinoléines analgésiques, anti-inflammatoires et antipyrétiques répondant à la formule générale:

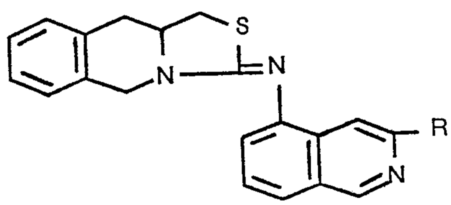

dans laquelle R est un radical alcoyle contenant 1 à 10 atomes de carbone.

La présente invention concerne de nouveaux dérivés de la thiazolo [3,4-b] isoquinoléine de formule générale:

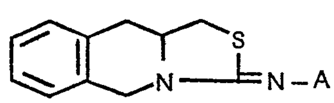 (I)

de forme (S), ou (R,S) ou leurs mélanges, leurs sels lorsqu'ils existent, leur préparation et les médicaments qui les contiennent.

Dans la formule générale (I), le symbole A représente un radical isoquinolyl-8, méthyl-3 isoquinolyl-8, hydroxyméthyl-3 isoquinolyl-5, carboxyméthyl-3 isoquinolyl-5, quinolyl-5, thiénopyridyle, benzimidazolyle, thiényle, thiazolyle, carboxyalcoyl-4 (ou -5) thiazolyl-2 dont la portion alcoyle est droite ou ramifiée et contient 1 à 4 atomes de carbone, thiadiazol-1,3,4 yl-2, pyrazolyle, imidazolyle, pyrimidinyle, pyridazinyle, ou pyrazinyle, les hétérocycles monocycliques ci-dessus pouvant être substitués par un radical alcoyle droit ou ramifié contenant 1 à 4 atomes de carbone.

Selon l'invention, les produits de formule générale (I) peuvent être obtenus par action de l'amine de formule générale:

$$A—NH_2 \qquad (II)$$

sur un sel de formule générale:

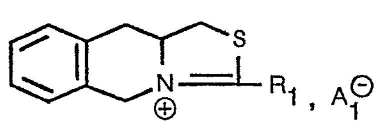 (III)

dans lesquelles A est défini selon l'invention, $R_1$ représente un atome de chlore ou un radical alcoylthio contenant 1 à 4 atomes de carbone ou benzylthio et $A_1$ représente un anion. On opère en milieu basique à une température de 15 à 50°C.

Lorsque $R_1$ représente un atome de chlore et $A_1^\ominus$ un ion chlorure, la réaction s'effectue dans un solvant organique tel que l'acétonitrile, en présence d'une base telle que la triéthylamine.

Lorsque $R_1$ représente un radical alcoylthio ou benzylthio et $A_1^\ominus$ un ion iodure, sulfate, tétrafluoroborate ou fluorosulfonate, la réaction s'effectue dans un solvant organique basique tel que la pyridine.

Le sel de formule générale (III) peut être préparé selon la méthode décrite dans BE - A - 844 927.

La préparation des amines de formule générale (II) s'effectue selon, ou par analogie avec, les méthodes décrites ci-après dans les exemples.

Selon l'invention les produits de formule générale (I) pour lesquels A est un radical isoquinolyl-8, méthyl-3 isoquinolyl-8, hydroxyméthyl-3 isoquinolyl-5, carboxyméthyl-3 isoquinolyl-5, thiénopyridyle, benzimidazolyl-6, quinolyl-5, pyrimidinyl-2 ou thiényl-2 (ces deux derniers étant éventuellement substitués par un radical alcoyle), peuvent également être obtenus par cyclisation d'une tétrahydro-1,2,3,4 isoquinoléine de formule générale:

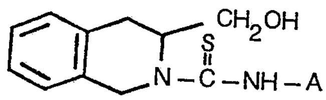

(IV)

dans laquelle A est défini comme ci-dessus.

La réaction s'effectue généralement par chauffage en milieu acide. Il est particulièrement avantageux d'opérer à une température comprise entre 65°C et la température de reflux du mélange réactionnel dans un acide minéral en solution aqueuse, par exemple dans l'acide chlorhydrique (de préférence 4N à 8N).

La tétrahydro-1,2,3,4 isoquinoléine de formule générale (IV) peut être obtenue par action d'un isothiocyanate de formule générale:

$$S{=}C{=}N{-}A \quad (V)$$

dans laquelle A est défini comme ci-dessus, sur l'hydroxyméthyl-3 tétrahydro-1,2,3,4 isoquinoléine.

Généralement la réaction s'effectue dans un solvant organique tel qu'un alcool, par exemple l'éthanol, en opérant à une température comprise entre 0 et 50°C.

L'hydroxyméthyl-3 tétrahydro-1,2,3,4 isoquinoléine peut être préparée à partir de la phénylalanine selon la méthode décrite par S. YAMADA et T. KUNIEDA, Chem. Pharm. Bull., *15,* 490 (1967).

Lorsque l'on utilise une phénylalanine de forme L, le produit de formule générale (I) est obtenu sous forme (S).

Lorsque l'on utilise une phénylalanine de forme DL, le produit de formule générale (I) est obtenu sous forme (RS).

L'isothiocyanate de formule générale (V) dans laquelle A est défini comme ci-dessus, à l'exception de représenter pyrimidinyl-2 ou thiényl-2 (éventuellement substitués par un radical alcoyle) peut être obtenu par condensation du sulfure de carbone avec l'amine de formule générale (II) dans laquelle A est défini comme ci-dessus, suivie de l'addition de dicyclohexylcarbodiimide.

La condensation s'effectue généralement en présence d'une base telle qu'une amine tertiaire, par exemple la triéthylamine. On opère avantageusement dans un solvant organique tel que la pyridine, à une température comprise entre −10 et 25°C.

L'isothiocyanato-2 pyrimidine (éventuellement substituée par un radical alcoyle) peut être préparée par la méthode décrite par W. ABRAHAM et G. BARNIKOW, Tetrahedron, *29,* 691 (1973).

L'isothiocyanato-2 thiophène (éventuellement substitué par un radical alcoyle) peut être préparé par la méthode décrite par W. C. McCARTHY et L. E. FOSS, J. Org. Chem. *42,* 1508 (1977).

Selon l'invention, le nouveau produit de formule générale (I) dans laquelle A est un radical hydroxyméthyl-3 isoquinolyl-5 peut être également obtenu par réduction d'un ester de formule générale:

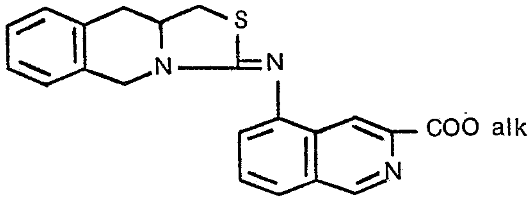

(VI)

dans laquelle alk est un radical alcoyle contenant 1 à 4 atomes de carbone.

La réduction s'effectue par toute méthode connue en soi pour réduire un ester en alcool. On opère notamment par action de l'hydrure de bore et de lithium préparé in situ, à une température comprise entre 0 et 20°C, dans un solvant organique tel que le diglyme ou par action de l'hydrure de bore et de calcium préparé in situ à une température voisine de 20°C, dans un solvant organique tel que le tétrahydrofuranne.

Selon l'invention, les nouveaux produits de formule générale (I) dans laquelle A représente un radical carboxyméthyl-3 isoquinolyl-5 ou carboxyalcoyl-4 (ou -5) thiazolyl-2 éventuellement substitué par un radical alcoyle peuvent être également obtenus à partir de l'ester correspondant de formule générale:

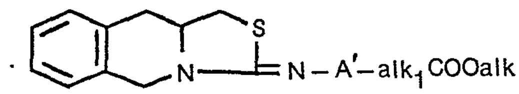

(VII)

(dans laquelle alk est défini comme précédemment, et soit —A' est un radical isoquinolyl-5 et $alk_1$ un radical méthylène en position -3, soit —A' est un radical thiazolyl-2 éventuellement substitué par un radical alcoyle et $alk_1$ est un radical alcoylène droit ou ramifié contenant 1 à 4 atomes de carbone, en position -4 ou -5) par toute méthode connue pour obtenir un acide à partir d'un ester sans toucher au reste de la molécule.

On peut opérer par hydrolyse acide ou alcaline, notamment par utilisation d'acide chlorhydrique à une température voisine de 100°C, de préférence dans l'acide aqueux 2 à 4N, ou bien par utilisation de soude ou de potasse à la température de reflux du mélange réactionnel, de préférence dans une solution aqueuse ou hydroalcoolique de soude de concentration 1 à 5 N.

Les produits de formules générales (VI) et (VII) peuvent être préparés par réaction des produits correspondants de formules (II) et (III), comme décrit ci-avant pour les produits de formule générale (I).

Lorsque A est autre que thiényle, les nouveaux produits selon l'invention peuvent être transformés en sels d'addition avec les acides. Les sels d'addition peuvent être obtenus par action du produit sur des acides dans des solvants appropriés; comme solvants organiques on utilise par exemple des alcools, des cétones, des éthers ou des solvants chlorés.

Lorsque A représente un radical carboxyméthyl-3 isoquinolyl-5 ou carboxyalcoyl-4 (ou -5) thiazolyl-2 éventuellement substitué par un radical alcoyle, les nouveaux produits selon l'invention peuvent être transformés en sels métalliques ou en sels d'addition avec une base azotée. Ces sels peuvent être obtenus par action d'une base métallique (notamment alcaline ou alcalinoterreuse), de l'ammoniac ou d'une base organique azotée, dans un solvant approprié tel qu'un alcool, un éther ou l'eau, ou par réaction d'échange avec un sel d'un acide organique.

Le sel formé précipite après concentration éventuelle de sa solution; il est séparé par filtration ou décantation.

On peut également obtenir le sel, à partir de sa solution, à l'état de lyophilisat.

Les nouveaux produits de formule générale (I) et/ou leurs sels lorsqu'ils existent peuvent être éventuellement purifiés par des méthodes physiques telles que la cristallisation ou la chromatographie.

Les nouveaux produits selon l'invention et leurs sels pharmaceutiquement acceptables présentent des propriétés pharmacologiques remanquables.

Ils présentent une activité antivirale qui s'exerce en particulier sur les virus du groupe rhinovirus.

Sur des cultures cellulaires de fibroblastes humains MRC—5 infectées avec le rhinovirus humain type 1B (souche R 1112) les produits selon l'invention provoquent l'inhibition complète de l'effet cytophathogène et de la multiplication des virus à des concentrations comprises entre 7 à 125$\mu$g/cm3 (concentration maximale non cytotoxique) et 0,016 à 30 $\mu$g/cm3 (concentration minimale inhibitrice).

Certains d'entre eux sont aussi des agents analgésiques, anti-thermiques et anti-inflammatoires intéressants.

L'activité anti-inflammatoire se manifeste chez le rat à des doses comprises entre 50 et 200 mg/kg par voie orale selon la technique de K. F. BENITZ et L. M. HALL, Arch. Int. Pharmacodyn. *144,* 185 (1963).

L'activité analgésique se manifeste chez le rat à des doses comprises entre 20 et 200 mg/kg par voie orale dans la technique de E. SIEGMUND et coll., Proc. Soc. Exp. Biol. Med. *95,* 729 (1957).

L'activité anti-thermique se manifeste chez le rat à des doses comprises entre 20 et 200 mg/kg par voie orale dans la technique de J. J. LOUX et coll., Toxicol. Appl. Pharmacol., *22,* 674 (1972).

Par ailleurs la toxicité aiguë des produits selon l'invention exprimée par leur $DL_{50}$ (dose létale 50%) est comprise entre 300 et 900 mg/kg ou supérieure à 900 mg/kg par voie orale chez la souris.

D'un intérêt particulier sont les produits de formule générale (I) dans laquelle A représente un radical isoquinolyl-8, méthyl-3 isoquinolyl-8, hydroxyméthyl-3 isoquinolyl-5, quinolyl-5, thiénopyridyl-3, benzimidazolyl-5, thiényl-2, thiazolyl-2, carboxyalcoyl-4 (ou -5) thiazolyl-2 (dont la portion alcoyle droite ou ramifiée contient 1 à 4 atomes de carbone, thiadiazol-1,3,4 yl-2, pyrazolyl-3, pyrimidinyl-2, pyridazinyl-3 ou pyrazinyl-2, les hétérocycles monocycliques ci-dessus pouvant être substitués par un radical alcoyle droit ou ramifié contenant 1 à 4 atomes de carbone.

Et parmi ces produits, plus spécialement actifs sont les produits de formule générale (I) dans laquelle A représente un radical isoquinolyl-8, méthyl-3 isoquinolyl-8, hydroxyméthyl-3 isoquinolyl-5, quinolyl-5, thiényl-2, thiazolyl-2, carboxylalcovl-4 thiazolyl-2 (dont la partie alcoyle contient 1 ou 2

atomes de carbone), pyrazolyl-3, pyrimidinyl-2 éventuellement substitué par un radical méthyle, pyridazinyl-3 ou pyrazinyl-2.

Pour l'emploi médicinal il peut être fait usage des nouveaux composés à l'état de sels pharmaceutiquement acceptables c'est-à-dire non toxiques aux doses d'utilisation.

Comme exemples de sels d'addition pharmaceutiquement acceptables peuvent être cités des sels d'acides minéraux (tels que chlorhydrates, sulfates, nitrates, phosphates) ou organiques (tels qu'acétates, propionates, succinates, benzoates, fumarates, maléates, tartrates, théophillineacétates, salicylates, phénolphtalinates, méthylène bis-$\beta$-oxynaphtoates ou dérivés de substitution de ces acides).

Comme exemples de sels métaux ou de sels d'addition avec les bases azotées peuvent être cités notamment les sels de sodium, potassium, calcium, lysine, éthanolamine.

Les exemples suivants, donnés à titre non limitatif, montrent comment l'invention peut être mise en pratique.

### Exemple 1

On fait passer un courant d'hydrogène sulfuré dans une solution de 10 g de tétrachlorostannate de bis (chlorhydrate d'amino-2-thiophène) dans 500 cm3 d'eau jusqu'à fin de précipitation de disulfure d'étain. L'insoluble est séparé par filtration. Le filtrat est débarassé de l'hydrogène sulfuré résiduel par un courant d'azote puis est ajouté, goutte à goutte, à une suspension de 13,7 g d'iodure de méthylthio-3 tétrahydro-1,5,10,10a thiazolo [3,4-b] isoquinoléinium-(S) dans 300 cm3 de pyridine. Après 17 heures d'agitation à une température voisine de 20°C, on concentre à sec sous pression réduite (25 mm de mercure; 3,3 kPa) à 60°C. Le résidu est dissous dans un mélange de 250 cm3 de soude 2N et de 250 cm3 de chlorure de méthylène.

La phase organique est décantée, séchée sur sulfate de magnésium, traitée au noir décolorant puis concentrée à sec sous pression réduite (40 mm de mercure; 5,3 kPa) à 40°C. Le résidu est dissous dans 100 cm3 d'acétonitrile bouillant; après refroidissement à 5°C, les cristaux formés sont séparés par filtration et lavés avec 10 cm3 d'acétonitrile glacé. Après séchage à 40°C sous pression réduite (1 mm de mercure; 0,13 kPa), on obtient 5,4 g de (thiényl-2 imino)-3-tétrahydro-1,5,10,10a thiazolo [3,4-b] isoquinoléine-(S) sous forme de cristaux blancs, F = 112°C.

$$[\alpha]_D^{20} = -\ 273 \pm 4° \text{ (c = 0,5; chloroforme)}$$

Le tétrachlorostannate de bis (chlorhydrate d'amino-2-thiophène) peut être préparé selon la méthode de W. STEINKOPF reproduite dans H. HARTOUGH, The Chemistry of Heterocyclic Compounds: Thiophene and its derivatives, Interscience Publishers, (1952), p. 513.

### Exemple 2

A une solution de 10,0 g d'amino-2 thiazole dans 500 cm3 de pyridine, on ajoute 18,2 g d'iodure de méthylthio-3 tétrahydro-1,5,10,10a thiazolo [3,4-b] isoquinoléinium-(S). La suspension passe progressivement en solution. Après 3 jours à une température voisine de 20°C, on concentre sous pression réduite (25 mm de mercure; 3,3 kPa); le résidu est dissous dans un mélange de 300 cm3 d'eau et de 300 cm3 de chlorure de méthylène. La phase organique est décantée, séchée sur sulfate de magnésium, filtrée puis concentrée à sec sous pression réduite (25 mm de mercure; 3,3 kPa). On ajoute au résidu 250 cm3 de propanol; on porte à ébullition et filtre à chaud. Après refroidissement, les cristaux formés sont séparés par filtration et lavés avec trois fois 10 cm3 de propanol. Après séchage à 40°C sous pression réduite (1 mm de mercure; 0,13 kPa), on obtient 11,9 g de produit, que l'on recristallise dans 250 cm3 d'acétonitrile. Après séchage à 60°C sous pression réduite (0,1 mm de mercure; (0,013 kPa), on obtient 10,6 g de (thiazolyl-2 imino)-3 tétrahydro-1,5,10,10a thiazolo [3,4-b] isoquinoléine-(S) forme de cristaux blancs, F = 158°C.

$$[\alpha]_D^{20} = -\ 235 \pm 2° \text{ (c = 2; chloroforme).}$$

### Exemple 3

En opérant comme à l'exemple 2 mais à partir de 18,2 g d'iodure de méthylthio-3 tétrahydro-1,5,10,10a thiazolo [3,4-b] isoquinoléinium-(S) et de 15,7 g d'amino-2 tert. butyl-5 thiadiazole-1,3,4, on obtient 7,0 g de (tert.butyl-5 thiadiazol-1,3,4 yl-2) imino-3 tétrahydro-1,5,10,10a thiazolo [3,4-b] isoquinoléine-(S) sous forme de cristaux blancs, F = 137°C.

$$[\alpha]_D^{20} = -\ 203 \pm 2° \text{ (c = 2, chloroforme)}$$

L'amino-2 tert. butyl-5 thiadiazole-1,3,4 peut être préparé selon la méthode de F. CHUBB, Cand. J. Chem., *37*, 1121 (1959).

Exemple 4

En opérant comme à l'exemple 2 mais à partir de 18,2 g d'iodure de méthylthio-3 tétrahydro-1,5,10,10a thiazolo [3,4-b] isoquinoléinium-(S) et de 8,3 g d'amino-3 pyrazole, on obtient 7,0 g de (pyrazolyl-3 imino)-3 tétrahydro-1,5,10,10a thiazolo [3,4-b] isoquinoléine-(S) sous forme de cristaux blancs, F= 161°C.

$$[\alpha]_D^{20} = -\ 256 \pm 3° \text{ (c = 1, chloroforme)}$$

Exemple 5

En opérant comme à l'exemple 2 mais à partir de 18,2 g d'iodure de méthylthio-3 tétrahydro-1,5,10,10a thiazolo [3,4-b] isoquinoléinium-(S) et de 8,0 g d'amino-2 méthyl-4 pyrimidine, on obtient 3,0 g de (méthyl-4 pyrimidinyl-2) imino -3 tétrahydro-1,5,10,10a thiazolo [3,4-b] isoquinoléine-(S) sous forme de cristaux blancs, F = 179°C.

$$[\alpha]_D^{20} = -\ 266 \pm 3° \text{ (c = 1; chloroforme)}$$

Exemple 6

En opérant comme à l'exemple 2, mais à partir de 18,2 g d'iodure de méthylthio-3 tétrahydro-1,5,10,10a thiazolo [3,4-b] isoquinoléinium-(S) et de 9,5 g d'amino-3 pyridazine, on obtient 9,3 g de (pyridazinyl-3 imino)-3 tétrahydro-1,5,10,10a thiazolo [3,4-b] isoquinoléine-(S) sous forme de cristaux beige clair, F = 140—142°C.

$$[\alpha]_D^{20} = -\ 276 \pm 3° \text{ (c = 2; chloroforme)}$$

Exemple 7

En opérant comme à l'exemple 2, mais à partir de 16,3 g d'iodure de méthylthio-3 tétrahydro-1,5,10,10a thiazolo [3,4-b] isoquinoléinium-(S) et de 8,5 g d'amino-2 pyrazine, on obtient 7,6 g de (pyrazinyl-2 imino)-3 tétrahydro-1,5,10,10a thiazolo [3,4-b] isoquinoléine-(S) sous forme de cristaux blancs, F = 140°C.

$$[\alpha]_D^{20} = -\ 283 \pm 3° \text{ (c = 1; chloroforme)}$$

Exemple 8

En opérant comme à l'exemple 2, mais à partir de 10,1 g d'iodure de méthylthio-3 tétrahydro-1,5,10,10a thiazolo [3,4-b] isoquinoléinium-(S) et de 6,4 g d'amino-3 thiéno [2,3-c] pyridine, on obtient 6,4 g de (thiéno [2,3-c] pyridyl-3) imino -3 tétrahydro-1,5,10,10a thiazolo [3,4-b] isoquinoléine-(S) sous forme de cristaux blancs, F = 180°C.

$$[\alpha]_D^{20} = -\ 205 \pm 3° \text{ (c = 1; chloroforme)}$$

L'amino-3 thiéno [2,3-c] pyridine peut être préparée selon la méthode de L.H. KLEMM, J. Het. Chem., *14*, 299 (1977).

Exemple 9

En opérant comme à l'exemple 2 mais à partir de 6,7 g d'iodure de méthylthio-3 tétrahydro-1,5,10,10a thiazolo [3,4-b] isoquinoléinium-(S) et de 2,8 g d'amino-3 thiéno [2,3-b] pyridine, on obtient 4,6 g de (thiéno [2,3-b] pyridyl-3) imino -3 tétrahydro-1,5,10,10a thiazolo [3,4-b] isoquinoléine-(S) sous forme de cristaux beiges, F = 145—146°C.

$$[\alpha]_D^{20} = -\ 204 \pm 3° \text{ (c = 0,5; chloroforme)}$$

L'amino-3 thiéno [2,3-b] pyridine peut être préparée selon la méthode de L.H. KLEMM et coll., J. Het. Chem. *7*, 373 (1970).

Exemple 10

En opérant comme à l'exemple 2 mais à partir de 18 g d'iodure de méthylthio-3 tétrahydro-1,5,10,10a thiazolo [3,4-b] isoquinoléinium-(S) et de 10 g de chlorhydrate d'amino-5 benzimidazole, on obtient 11,7 g de [benzimidazolyl-5 (ou -6) imino]-3 tétrahydro-1,5,10,10a thiazolo [3,4-b] isoquinoléine-(S) sous forme de cristaux roses, F = 220°C.

$$[\alpha]_D^{20} = -\ 236 \pm 3° \text{ (c = 1; méthanol)}$$

Le chlorhydrate de l'amino-5 benzimidazole peut être préparé selon la méthode de D. WOOLLEY, J. Biol. Chem., *152*, 225 (1944).

Exemple 11

En opérant comme à l'exemple 2 mais à partir de 18,2 g d'iodure de méthylthio-3 tétrahydro-1,5,10,10a thiazolo [3,4-b] isoquinoléinium-(S) et de 10,9 g d'amino-8 isoquinoléine, on obtient 11,8 g d'(isoquinolyl-8 imino)-3 tétrahydro-1,5,10,10a thiazolo [3,4-b] isoquinoléine-(S) sous forme de cristaux jaune pâle, F = 206°C.

$$[\alpha]_D^{20} = -197 \pm 2° \text{ (c = 1; chloroforme)}$$

L'amino-8 isoquinoléine peut être préparée selon la méthode de D.H. HEY, J. Chem. Soc., 3882 (1961).

Exemple 12

En opérant comme à l'exemple 2, mais à partir de 8 g d'iodure de méthylthio-3 tétrahydro-1,5,10,10a thiazolo [3,4-b] isoquinoléinium-(S) et de 3,5 g d'amino-8 méthyl-3 isoquinoléine, on obtient 4,1 g de (méthyl-3 isoquinolyl-8) imino -3 tétrahydro-1,5,10,10a thiazolo [3,4-b] isoquinoléine-(S) sous forme de cristaux jaune pâle, F= 156°C.

$$[\alpha]_D^{20} = -186° \pm 3° \text{ (c = 1; chloroforme)}$$

L'amino-8 méthyl-3 isoquinoléine peut être préparée de la façon suivante:

A une solution de 10,8 g de bromo-5 méthyl-3 nitro-8 isoquinoléine dans 200 cm3 d'éthanol on ajoute 5 g de catalyseur constitué de palladium sur carbone (3—97 en poids). On agite cette suspension en chauffant à reflux sous atmosphère d'azote, et on ajoute goutte à goutte, en 30 minutes, 25 cm3 d'hydrate d'hydrazine. Après la fin de l'addition on chauffe encore pendant 2 heures à reflux. La suspension est filtrée à chaud sur silice de diatomées et le filtrat est concentré à sec sous pression réduite (20 mm de mercure; 2,7 kPa) à 40°C. Le résidu est dissous dans 200 cm3 de solution aqueuse 0,5 N d'acide chlorhydrique et on extrait avec deux fois 50 cm3 de chlorure de méthylène. La phase aqueuse est ensuite alcalinisée à pH 10 par addition de soude et extraite par trois fois 150 cm3 de chlorure de méthylène. Les extraits organiques sont réunis, lavés à l'eau, séchés sur sulfate de magnésium, filtrés et concentrés à sec sous pression réduite (20 mm de mercure; 2,7 kPa) à 40°C. On obtient 4,4 g d'amino-8 méthyl-3 isoquinoléine, solide beige, F = 175°C.

La bromo-5 méthyl-3 nitro-8 isoquinoléine peut être préparée de la façon suivante:

On dissout 17,2 g de bromo-5 méthyl-3 isoquinoléine dans 100 cm3 d'acide sulfurique (densité 1,83; 34N). On agite cette solution et ajoute en 30 minutes 8,1 g de nitrate de potassium en maintenant la température entre 0 et 5°C par un bain de glace, puis on laisse la température remonter aux environs de 20°C. On agite encore pendant 5 heures à cette température puis on verse la solution dans 600 cm3 de mélange d'eau et de glace et ajoute de l'ammoniaque à 20% de $NH_3$ (densité 0,9) jusqu'à obtention de pH 10 sans dépasser 30°C. La précipité jaune obtenu est séparé par filtration, lavé à l'eau, séché et recristallisé dans 300 cm3 d'éthanol.

Après filtration et séchage, on obtient 12,3 g de bromo-5 méthyl-3 nitro-8 isoquinoléine sous forme d'un solide beige, F = 146°C.

La bromo-5 méthyl-3 isoquinoléine peut être préparée par la méthode décrite par M. GORDON et coll., J. Het. Chem., *4*, 410 (1967).

Exemple 13

En opérant comme à l'exemple 2, mais à partir de 18,2 g d'iodure de méthylthio-3 tétrahydro-1,5,10,10a thiazolo [3,4-b] isoquinoléinium-(S) et de 14,4 g d'amino-5 quinoléine, on obtient 15,0 g de (quinolyl-5 imino)-3 tétrahydro-1,5,10,10a thiazolo [3,4-b] isoquinoléine-(S) sous forme de cristaux beige clair, F = 194°C.

$$[\alpha]_D^{20} = -195 \pm 2° \text{ (c = 2; chloroforme)}$$

Exemple 14

On mélange à 0°C 1,01 g de chlorure de lithium, 0,91 g de borohydrure de sodium et 100 cm3 de diglyme (éther diméthylique du diéthylèneglycol). On agite pendant 30 minutes à 0°C et ajoute 4,7 g de [(méthoxycarbonyl-3 isoquinolyl-5) imino]-3 tétrahydro-1,5,10,10a thiazolo [3,4-b] isoquinoléine-(S).On poursuit l'agitation pendant 1 heure à 0°C puis 2 heures à 20°C. On ajoute 1 litre d'eau et extrait avec trois fois 150 cm3 de chlorure de méthylène. Les extraits organiques sont réunis, lavés à l'eau, séchés sur sulfate de magnésium, filtrés et concentrés à sec sous pression réduite (40 mm de mercure; 5,3 kPa) à 40°C. Le résidue est dissous dans 20 cm3 de chlorure de méthylène et la solution est versée sur une colonne de 3 cm de diamètre contenant 120 g de gel de silice dans du chlorure de méthylène. On élue par un mélange d'acétate d'éthyle et de cyclohexane (70—30 en volumes) en collectant des fractions de 300 cm3. Les fractions 7 à 11 sont réunies et concentrées à sec sous pression réduite (20 mm de mercure; 2,7 kPa) à 40°C. Le résidue est recristallisé dans 100 cm3 d'acétate de butyle. Après séparation sur filtre, lavage à l'oxyde d'isopropyle et séchage on

obtient 2 g d'(hydroxyméthyl-3 isoquinolyl-5) imino -3 tétrahydro-1,5,10,10a thiazolo [3,4-b] isoquinoléine-(S), solide blanc, F = 210°C.

$$[\alpha]_D^{20} = -172 \pm 3° \text{ (c = 0,5; chloroforme)}$$

La (méthoxycarbonyl-3 isoquinolyl-5) imino -3 tétrahydro-1,5,10,10a isoquinoléine-(S) peut être préparée de la façon suivante:

A une solution de 13 g d'amino-5 méthoxycarbonyl-3 isoquinoléine dans 400 cm3 de pyridine on ajoute 22,4 g d'iodure de méthylthio-3 tétrahydro-1,5,10,10a thiazolo [3,4-b] isoquinoléinium-(S). La suspension passe progressivement en solution. Après 3 jours d'agitation à une température voisine de 20°C on concentre à sec sous pression réduite (20 mm de mercure; 2,7 kPa) à 60°C. Le résidu est dissous dans 500 cm3 de chlorure de méthylène et lavé avec trois fois 300 cm3 de solution aqueuse N de soude, puis à l'eau. Après séchage sur sulfate de sodium et filtration on concentre à sec sous pression réduite (20 mm de mercure; 2,7 kPa) à 40°C. Le résidu est recristallisé dans 500 cm3 d'acétonitrile. Après séparation sur filtre, lavage à l'oxyde d'isopropyle et séchage on obtient 12,8 g de (méthoxy-carbonyl-3 isoquinolyl-5) imino -3 tétrahydro-1,5,10,10a isoquinoléine-(S), solide jaune pâle, F = 200°C.

L'amino-5 méthoxycarbonyl-3 isoquinoléine peut être préparée de la façon suivante:

On ajoute 25 g d'acide amino-5 isoquinoléinecarboxylique-3 à 2 litres de méthanol. On chauffe à reflux et fait barboter pendant 3 heures un courant d'acide chlorhdyrique gazeux sec. On chauffe encore pendant 3 heures à reflux, puis concentre à sec sous pression réduite (20 mm de mercure; 2,7 kPa) à 60°C. Le résidu est dissous dans 200 cm3 d'eau et lavé avec deux fois 100 cm3 d'acétate d'éthyle. La solution aqueuse est alcalinisée à pH 9 par addition de carbonate de potassium. Le précipité formé est séparé par filtration, lavé à l'eau et séché. On obtient 20,3 g d'amino-5 méthoxycarbonyl-3 isoquinoléine.

L'acide amino-5 isoquinoléinecarboxylique-3 peut être préparé de la façon suivante:

On dissout 44 g d'acide nitro-5 isoquinoléinecarboxylique-3 dans 3 litres de méthanol et ajoute 15 g de catalyseur constitué de palladium sur carbone (3—97 en poids). On hydrogène à la pression atmosphérique à une température comprise entre 20 et 25°C. Après 5 heures d'agitation, le volume d'hydrogène absorbé est de 13,9 litres. On filtre sur silice de diatomées et le filtrat est concentré à sec sous pression réduite (20 mm) de mercure; 2,7 kPa) à 60°C. On obtient 35 g d'acide amino-5 isoquinoléinecarboxylique-3.

L'acide nitro-5 isoquinoléinecarboxylique peut être préparé par la méthode décrite par R.C. ELDERFIELD et coll., J. Org. Chem., *23*, 435 (1958).

Exemple 15

On chauffe à 100°C pendant 6 heures, une solution de 3,8 g d'(éthoxycarbonylméthyl-3 isoquinolyl-5) imino -3 tétrahydro-1,5,10,10a thiazolo [3,4-b] isoquinoléine-(S) dans 60 cm3 d'acide chlorhydrique 3N. Après refroidissement, on ajuste le pH de la solution à 7 par addition de soude 5 N, puis on extrait avec 2 fois 200 cm3 de chlorure de méthylène. Les extraits organiques sont réunis, séchés sur sulfate de magnésium puis concentrés à sec. Le résidu obtenu est dissous dans 60 cm3 d'un mélange de diméthylformamide et d'eau (2:1 en volumes) porté à ébullition, on ajoute 0,3 g de noir décolorant et on filtre à chaud. Après refroidissement à 5°C pendant 20 heures, les cristaux apparus sont séparés par filtration, lavés avec de l'oxyde d'isopropyle puis séchés à 70°C sous pression réduite (0,1 mm de mercure; 0,013 kPa). On obtient ainsi 1,7 g de (carboxyméthyl-3 isoquinolyl-5) imino -3 tétrahydro-1,5,10,10a thiazolo [3,4-b] isoquinoléine-(S) fondant vers 200°C (avec décomposition).

$$[\alpha]_D^{20} = -152 \pm 2° \text{ (C = 1; chloroforme)}$$

L'(éthoxycarbonylméthyl-3 isoquinolyl-5) imino -3 tétrahydro-1,5,10,10a thiazolo [3,4-b] isoquinoléine-(S) peut être préparée de la façon suivante:

A une solution de 15,8 g d'amino-5 isoquinoléineacétate-3 d'éthyle dans 200 cm3 de pyridine, on ajoute 18,5 g d'iodure de méthylthio-3 tétrahydro-1,5,10,10a thiazolo [3,4-b] isoquinoléinium-(S). Après 15 heures à une température voisine de 20°C, la solution est concentrée sous pression réduite (25 mm de mercure; 3,3 kPa). Le résidu est dissous dans un mélange de 2 litres de chlorure de méthylène et de 1 litre de soude 5 N. La phase organique est décantée, lavée avec 3 fois 1 litre d'eau, séchée sur sulfate de sodium puis concentrée à environ 100 cm3, sous pression réduite (25 mm de mercure; 3,3 kPa).

On verse cette solution sur une colonne de 600 g de gel de silice (diamètre de la colonne: 5,5 cm) puis on élue avec du chlorure de méthylène en recueillant des fractions d'éluat de 500 cm3. On évapore les fractions 18 à 24; le résidu est cristallisé dans un mélange acétonitrile-éther (10—50 en volumes). On obtient ainsi 9,1 g d'(éthoxycarbonylméthyl-3 isoquinolyl-5) imino -3 tétrahydro-1,5,10,10a thiazolo [3,4-b] isoquinoléine-(S) sous forme de cristaux jaunes; F = 112°C.

$$[\alpha]_D^{20} = -146 \pm 2° \text{ (c = 1; chloroforme)}$$

L'amino-5 isoquinoléineacétate-3 d'éthyle peut être préparé de la façon suivante:

On dissout 20 g de nitro-5 isoquinoléineacétate-3 d'éthyle dans 1 litre d'acétate d'éthyle et ajoute 21 g de catalyseur constitué de palladium sur carbone (3—97 en poids). On hydrogène à la pression atmosphérique à une température voisine de 50°C pendant 2 heures. On filtre sur silice de diatomées puis concentre le filtrat à sec sous pression réduite (20 mm de mercure; 2,7 kPa) à 50°C. On obtient ainsi 15,8 g d'amino-5 isoquinoléineacétate-3 d'éthyle sous forme de cristaux beiges, F = 90°C.

Le nitro-5 isoquinoléineacétate-3 d'éthyle peut être préparé de la façon suivante:

On verse goutte à goutte une solution de 300 cm3 de chlorure de méthylène contenant 21,5 g d'isoquinoléineacétate-3 d'éthyle dans une suspension de 1700 cm3 de chlorure de méthylène refroidie à 10°C, contenant du trifluorométhanesulfonate de nitronium préparé in situ par addition successive de 70 cm3 d'acide trifluorométhanesulfonique et de 15,1 cm3 d'acide nitrique (d = 1,52; 23 N). On maintient le milieu réactionnel à 10°C pendant 2 heures puis on le laisse se réchauffer jusqu'à une température voisine de 20°C.

On neutralise par addition d'une solution de carbonate de sodium sous agitation. La phase organique est décantée, lavée avec 3 fois 1 liter d'eau, séchée sur sulfate de sodium puis concentrée à sec. On effectue une purification par chromatographie sur 400 g de gel de silice contenus dans une colonne de 4,5 cm de diamètre. On élue avec, successivement, 2 litres de chlorure de méthylène, 2 litres de mélange chlorure de méthylène-méthanol (98—2 en volumes) et 4 litres de mélange chlorure de méthylène-méthanol (96—4 en volumes) en recueillant l'éluat par fractions de 800 cm3. Après évaporation à sec des fractions 7 à 9, on obtient 20 g de nitro-5 isoquinoléine-acétate-3 d'éthyle sous forme de cristaux bruns fondant vers 90°C.

L'isoquinoléineacétate-3 d'éthyle peut être préparé selon la méthode de P. CROOKS, J. Med. Chem., *21* 585 (1978).

## Exemple 16

A une solution de 7,9 g d'[(éthoxycarbonylméthyl-5 thiazolyl-2) imino]-3 tétrahydro-1,5,10,10a thiazolo [3,4-b] isoquinoléine-(S) dans 60 cm3 d'éthanol on ajoute 20 cm3 de solution aqueuse 5N de soude. On chauffe à reflux pendant deux heures. Après refroidissement à 20°C on acidifie par addition de 10 cm3 d'acide chlorhydrique 12 N (d = 1,19). Le précipité obtenu est filtré, lavé avec 3 fois 50 cm3 d'eau, puis 50 cm3 d'éthanol et 20 cm3 d'éther éthylique. Le produit est recristallisé par dissolution dans 15 cm3 de diméthylformamide à 70°C, addition de 80 cm3 d'éthanol et refroidissement à 0°C. Les cristaux obtenus sont filtrés, lavés avec 50 cm3 d'éthanol, puis 20 cm3 d'éther éthylique et séchés à 50°C sous pression réduite (0,1 mm de mercure; 0,013 kPa). On obtient ainsi 6,8 g de chlorhydrate de [(carboxyméthyl-5 thiazolyl-2) imino]-3 tétrahydro-1,5,10,10a thiazolo [3,4-b] isoquinoléine-(S), fondant vers 240°C.

$$[\alpha]_D^{20} = 301° \pm 4° \text{ (c = 0,5; méthanol)}$$

L'[(éthoxycarbonylméthyl-5 thiazolyl-2) imino]-3 tétrahydro-1,5,10,10a thiazolo [3,4-b] isoquinoléine-(S) peut être préparée de la façon suivante:

A une solution de 5,1 g d'(amino-2 thiazolyl-5) acétate d'éthyle dans 150 cm3 de pyridine on ajoute 10 g d'iodure de méthylthio-3 tétrahydro-1,5,10,10a thiazolo [3,4-b] isoquinoléinium-(S). Après 48 heures à une température voisine de 20°C, la solution est concentrée à sec sous pression réduite (25 mm de mercure; 3,3 kPa). Le résidu est dissous dans un mélange de 200 cm3 de chlorure de méthylène et 100 cm3 de soude N. La phase organique est décantée, lavée avec 3 fois 50 cm3 d'eau, séchée sur sulfate de sodium, puis concentrée à sec sous pression réduite (25 mm de mercure; 3,3 kPa) à 40°C. Le résidu est cristallisé par dissolution dans 100 cm3 d'éthanol bouillant et refroidissement à 0°C. Les cristaux obtenus sont filtrés, lavés avec 30 cm3 d'éthanol et 20 cm3 d'éther éthylique et séchés à 20°C pression réduite (0,1 mm de mercure; 0,013 kPa). On obtient ainsi 7,9 g d'[(éthoxycarbonylméthyl-5 thiazolyl-2) imino]-3 tétrahydro-1,5,10,10a thiazolo [3,4-b] isoquinoléine-(S), F = 132°C.

L'(amino-2 thiazolyl-5) acétate d'éthyle peut être préparé de la façon suivante:

On mélange 14,5 g de thiourée et 39,8 g de bromo-3 formyl-3 propionate d'éthyle dans 250 cm3 d'éthanol. On agite pendant 20 heures à une température voisine de 20°C. On observe la dissolution progressive des réactifs. La solution est concentrée à sec sous pression réduite (25 mm de mercure; 3,3 kPa) à 40°C. Le résidu est dessous dans un mélange de 200 cm3 de chlorure de méthylène et 200 cm3 de soude N. La phase organique est lavée à l'eau, séchée sur sulfate de magnésium, filtrée et concèntrée à sec sous pression réduite (25 mm de mercure; 3,3 kPa) à 40°C. Le résidu est cristallisé par dissolution dans 150 cm3 d'eau bouillante en présence de 3 g de charbon actif, filtration á chaud et refroidissement à 5°C. Les cristaux obtenus sonte filtrés, lavés à l'eau et séchés à 20°C sous pression réduite (0,1 mm de mercure, 0,013 kPa) en présence de $P_2O_5$. On obtient 7,2 g d'(amino-2 thiazolyl-5) acétate d'éthyle, F= 101°C.

Le bromo-3 formyl-3 propionate d'éthyle peut être préparé par la méthode de M. AEBERLI et H. ERLENMEYER, Helv. Ch. Acta. *33*, 503 (1950).

Exemple 17

A une solution de 10 g d'[(éthoxycarbonylméthyl-4 thiazolyl-2) imino]-3 tétrahydro-1,5,10,10a thiazolo [3,4-b] isoquinoléine-(S) dans 60 $cm^3$ d'éthanol on ajoute 30 cm3 de solution aqueuse 4 N de soude. On chauffe à reflux pendant deux heures. Après refroidissement à 20°C on acidifie par addition de 11 cm3 d'acide chlorhydrique 12 N (d = 1,19). Le précipité blanc qui s'est formé est filtré, lavé à l'eau, séché à 20°C sous pression réduite (0,1 mm mercure; 0,013 kPa). Le solide obtenu est cristallisé par dissolution dans 200 cm3 d'éthanol bouillant, refroidissement et filtration des cristaux formés; puis le produit est recristallisé par dissolution dans 60 cm3 de diméthylformamide à 100°C, addition de 120 cm3 d'éthanol et refroidissement à 0°C. Les cristaux sont filtrés, lavés avec 20 cm3 d'éthanol puis 20 cm3 d'éther éthylique et séchés à 40°C sous pression réduite (0,1 mm de mercure; 0,013 kPa). On obtient 5,6 g de chlorhydrate de [(carboxyméthyl-4 thiazolyl-2) imino]-3 tétrahydro-1,5,10,10a thiazolo [3,4-b] isoquinoléine-(S), F = 206°C.

$$[\alpha]_D^{20} = -267° \pm 3° \text{ (c = 1; méthanol)}$$

Spectre UV (méthanol): $\lambda$ max =305 nm ($\varepsilon$ = 16370)

A une solution de 0,5 g de chlorhydrate de [(carboxyméthyl-4 thiazolyl-2) imino]-3 tétrahydro-1,5,10,10a thiazolo [3,4-b] isoquinoléine-(S) dans 30 cm3 de méthanol on ajoute 0,8 g d'oxyde de propylène. On agite pendant 16 heures à une température voisine de 20°C. On filtre le précipité qui s'est formé et on le sèche sous pression réduite (20 mm de mercure; 2,7 kPa) à 40°C. On obtient 0,2 g de [(carboxyméthyl-4 thiazolyl-2) imino]-3 tétrahydro-1,5,10,10a thiazolo [3,4-b] isoquinoléine-(S), solide blanc F: 185°C.

Le sel de sodium de [(carboxyméthyl-4 thiazolyl-2) imino]-3 tétrahydro-1,5,10,10a thiazolo [3,4-b] isoquinoléine-(S) peut être préparé de la façon suivante:

A une suspension agitée de 0,17 g de [(carboxyméthyl-4 thiazolyl-2) imino]-3 tétrahydro-1,5,10,10a thiazolo [3,4-b] isoquinoléine-(S) dans 10 cm3 d'eau on ajoute 5 cm3 de soude 0,1 N. La solution obtenue est filtrée pour éliminer un léger insoluble et concentrée à sec sous pression réduite (20 mm de mercure; 2,7 kPa) à 60°C. Le solide blanc obtenu est séché sous pression réduite (0,1 mm de mercure; 0,013 kPa) à 60°C. On obtient 0,17 g de sel de sodium de la [(carboxyméthyl-4 thiazolyl-2) imino]-3 tétrahydro-1,5,10,10a, thiazolo [3,4-b] isoquinoléine-(S).

Spectre U.V (eau): $\lambda$ max =305 nm ($\varepsilon$ = 12300)

L'[(éthoxycarbonylméthyl-4 thiazolyl-2) imino]-3 tétrahydro-1,5,10,10a thiazolo [3,4-b] isoquinoléine-(S) peut être préparée de la façon suivante:

A une solution de 10 g d'(amino-2 thiazolyl-4) acétate d'éthyle dans 300 cm3 de pyridine, on ajoute 18,1 g d'iodure de méthylthio-3 tétrahydro-1,5,10,10a thiazolo [3,4-b] isoquinoléinium-(S). Après 48 heures à une température voisine de 20°C, la solution est concentrée à sec sous pression réduite (25 mm de mercure; 3,3 kPa) à 60°C. Le résidu est dissous dans un mélange de 200 cm3 de chlorure de méthylène et de 100 cm3 de soude N. La phase organique est décantée, lavée avec 3 fois 100 cm3 d'eau, séchée sur sulfate de sodium puis concentrée à sec sous pression réduite (25 mm de mercure; 3,3 kPa) à 40°C. Le résidu est recristallisé par dissolution dans 100 cm3 d'acétonitrile bouillant puis refroidissement. Les cristaux formés sont filtrés, lavés avec 20 cm3 d'acétonitrile puis 20 cm3 d'éther éthylique et séchés à 20°C sous pression réduite (0,1 mm de mercure; 0,013 kPa). On obtient ainsi 11,7 g d'[(éthoxycarbonylméthyl-4 thiazolyl-2) imino]-3 tétrahydro-1,5,10,10a thiazolo [3,4-b] isoquinoléine-(S), F= 105°C.

L'(amino-2 thiazolyl-4) acétate d'éthyle peut être préparé selon la méthode décrite par M. STEUDE, Liebigs Ann. Chem., *261* 22 (1891).

Exemple 18

A une solution de 7,4 g de {[(méthoxycarbonyl-4 butyl)-4 thiazolyl-2] imino}-3 tetrahydro-1,5,10,10a thiazolo [3,4-b] isoquinoléine-(S) dans 60 cm3 d'éthanol on ajoute 20 cm3 de solution aqueuse 5N de soude. On chauffe à reflux pendant 2 heures. Après refroidissement à 20°C on acidifie par addition de 10 cm3 d'acide chlorhydrique 12N (d = 1,19). On ajoute 30 cm3 d'éther éthylique et refroidit à 0°C. Le précipité obtenu est filtré, lavé avec 4 fois 15 cm3 d'eau, 2 fois 10 cm3 d'éthanol et 2 fois 30 cm3 d'éther éthylique, et cristallisé par dissolution dans 200 cm3 de propanol-2 bouillant et refroidissement à 0°C. Les cristaux obtenus sont filtrés, lavés avec 20 cm3 de propanol-2 et 50 cm3 d'éther éthylique, et séchés à 45°C sous pression réduite (0,1 mm de mercure; 0,013 kPa). On obtient ainsi 4,9 g de chlorhydrate de {[(carboxy-4-butyl)-4 thiazolyl-2] imino}-3 tétrahydro-1,5,10,10a thiazolo [3,4-b] isoquinoléine-(S), F = 214°C.

$$[\alpha]_D^{20} = -226° \pm 3° \text{ (c = 1; méthanol)}$$

La {[(méthoxycarbonyl-4 butyl)-4 thiazolyl-2] imino}-3 tétrahydro-1,5,10,10a thiazolo [3,4-b] isoquinoléine-(S) peut être obtenue de la façon suivante:

A une solution de 5,9 g d'(amino-2 thiazolyl-4)-5 valérate de méthyle dans 150 cm3 de pyridine on ajoute 10 g d'iodure de méthylthio-3 tétrahydro-1,5,10,10a thiazolo [3,4-b] isoquinoléinium-(S).

Après 48 heures à une température voisine de 20°C, la solution est concentrée à sec sous pression réduite (25 mm de mercure, 3,3 kPa) à 60°C. Le résidu est agité dans un mélange de 250 cm3 de chlorure de méthylène et 100 cm3 de soude N.

Le produit insoluble est filtré, lavé à l'eau puis à l'éthanol et recristallisé par dissolution dans 100 cm3 d'éthanol bouillant et refroidissement à 0°C. Les cristaux obtenus sont filtrés, lavés avec 20 cm3 d'éthanol et 20 cm3 d'éther éthylique et séchés à 20°C sous pression réduite (0,1 mm de mercure; 0,013 kPa). On obtient 7,4 g de {[(méthoxycarbonyl-4 butyl)-4 thiazolyl-2] imino}-3 tétrahydro-1,5,10,10a thiazolo [3,4-b] isoquinoléine-(S), solide blanc, F = 92°C.

L'(amino-2 thiazolyl-4)-5 valérate de méthyle peut être préparé de la façon suivante:

On mélange 15,7 g de thiourée et 39,7 g de chloro-7 oxo-6 heptanoate de méthyle dans 200 cm3 d'éthanol. On agite pendant 20 heures à une température voisine de 20°C: on observe la dissolution progressive des réactifs. La solution est concentrée à sec sous pression réduite (25 mm de mercure; 3,3 kPa) à 40°C. Le résidu est agité dans un mélange de 250 cm3 de chlorure de méthylène et 250 cm3 de soude N. Le produit insoluble est filtré, lavé à l'eau et recristallisé par dissolution dans 350 cm3 d'eau bouillante et refroidissement à 5°C. Les cristaux obtenus sont lavés avec 3 fois 30 cm3 d'eau, 3 fois 30 cm3 d'éthanol et 2 fois 50 cm3 d'éther éthylique et séchés à 20°C sous pression réduite (0,1 mm de mercure; 0,013 kPa). On obtient 31 g d'(amino-2 thiazolyl-4)-5 valérate de méthyle, solide blanc, F = 131°C.

Le chloro-7 oxo-6 heptanoate de méthyle peut être préparé selon la méthode décrite dans BE—A—867 128.

Exemple 19

A une solution de 8,2 g d'{[(éthoxycarbonyl-1 éthyl-1)-4 thiazolyl-2] iminio}-3 tétrahydro-1,5,10,10a thiazolo [3,4-b] isoquinoléine-(10a S) dans 60 cm3 d'éthanol on ajoute 20 cm3 de solution aqueuse 5 N de soude. On chauffe à reflux pendant deux heures. Après refroidissement à 20°C on acidifie par addition de 10 cm3 d'acide chlorhydrique 12 N (d = 1,19). On ajoute 30 cm3 d'éther éthylique et refroidit à 0°C. Le précipité obtenu est filtré, lavé à l'eau puis à l'éthanol et recristallisé dans 80 cm3 d'éthanol bouillant. Les cristaux obtenus après refroidissement sont lavés à l'éthanol et séchés à 50°C sous pression réduite (0,1 mm de mercure; 0,013 kPa). On obtient ainsi 3,8 g de chlorhydrate de {[(carboxy-1 éthyl-1)-4 thiazolyl-2] imino}-3 tétrahydro-1,5,10,10a thiazolo [3,4-b] isoquinoléinium-(10a S), fondant vers 200°—210°C.

$$[\alpha]_D^{20} = -234° \pm 3° \text{ (c = 0,5; méthanol)}$$

L'{[(éthoxycarbonyl-1 éthyl-1)-4 thiazolyl-2] imino}-3 tétrahydro-1,5,10,10a thiazolo [3,4,-b] isoquinoléine-(10a S) peut être préparée de la façon suivante:

A une solution de 5,5 g d'(amino-2 thiazolyl-4)-2 propionate d'éthyle dans 150 cm3 de pyridine on ajoute 10 g d'iodure de méthylthio-3 tétrahydro-1,5,10,10a thiazolo [3,4-b] isoquinoléinium-(S). Après 48 heures à une température voisine de 20°C, la solution est concentrée à sec sous pression réduite (25 mm de mercure; 3,3 kPa). Le résidu est dissous dans un mélange de 100 cm3 de chlorure de méthylène et de 100 cm3 de soude N. La phase organique est décantée, lavée avec 3 fois 50 cm3 d'eau, séchée sur sulfate de sodium puis concentrée à sec sous pression réduite (25 mm de mercure; 3,3 kPa) à 40°C. On obtient 8,2 g d'{[(éthoxycarbonyl-1 éthyl-1)-4 thiazolyl-2] imino}-3 tétrahydro-1,5,10,10a thiazolo [3,4-b] isoquinoléine-(10a S) sous forme d'une huile jaune.

L'(amino-2 thiazolyl-4)-2 propionate d'éthyle peut être préparé par la méthode décrite par R.G. WOODBRIDGE et G. DOUGHERTY, J. Am. Chem. Soc., *71*, 1744 (1949).

Exemple 20

A une solution de 8,1 g de [(méthoxycarbonylméthyl-5-méthyl-4 thiazolyl-2) imino]-3 tétrahydro-1,5,10,10a thiazolo [3,4-b] isoquinoléine-(S) dans 60 cm3 d'éthanol on ajoute 20 cm3 d'une solution aqueuse 5 N de soude. On chauffe à reflux pendant deux heures. Après refroidissement à 20°C on acidifie par addition de 10 cm3 d'acide chlorhydrique 12 N (d = 1,19). Le précipité obtenu est filtré, lavé avec 5 fois 25 cm3 d'eau, 3 fois 15 cm3 d'éthanol et 2 fois 25 cm3 d'éther éthylique et recristallisé par dissolution dans 80 cm3 de diméthylformamide à 150°C et refroidissement à 0°C. Les cristaux obtenus sont filtrés, lavés avec 10 cm3 de diméthylformamide, 4 fois 15 cm3 d'éthanol et 3 fois 15 cm3 d'éther éthylique et séchés à 45°C sous pression réduite (0,1 mm de mercure; 0,013 kPa). On obtient ainsi 4,8 g de chlorhydrate de [(carboxyméthyl-5 méthyl-4 thiazolyl-2) imino]-3 tétrahydro-1,5,10,10a thiazolo [3,4-b] isoquinoléine-(S), solide blanc fondant vers 250°C.

$$[\alpha]_D^{20} = -293° \pm 3° \text{ (c = 0,5; méthanol).}$$

La [(méthoxycarbonylméthyl-5 méthyl-4 thiazolyl-2) imino]-3 tétrahydro-1,5,10,10a thiazolo [3,4-b] isoquinoléine-(S) peut être obtenue de la façon suivante:

A une solution de 5,5 g d'(amino-2 méthyl-4 thiazolyl-5) acétate d'éthyle dans 150 cm3 de pyridine on ajoute 10 g d'iodure de méthylthio-3 tétrahydro-1,5,10,10a thiazolo [3,4-b]

isoquinoléinium-(S). Après 48 heures à une température voisine de 20°C, la solution est concentrée à sec sous pression réduite (25 mm de mercure; 3,3 kPa) à 60°C. Le résidu est dissous dans un mélange de 200 cm3 de chlorure de méthylène et 100 cm3 de soude N. La phase organique est lavée avec 2 fois 100 cm3 d'eau, séchée sur sulfate de magnésium, filtrée et concentrée à sec sous pression réduite (25 mm de mercure; 3,3 Kpa) à 40°C. Le résidu est cristallisé par dissolution dans 120 cm3 d'éthanol bouillant et refroidissement à 0°C. Les cristaux obtenus sont filtrés, lavés avec 30 cm3 d'éthanol et 20 cm3 d'éther éthylique et séchés à 20°C sous pression réduite (0,1 mm de mercure; 0,013 kPa). On obtient ainsi 8,1 g de [(méthoxycarbonylméthyl-5 méthyl-4 thiazolyl-2) imino]-3 tetrahydro-1,5,10,10a thiazolo [3,4-b] isoquinoléine-(S), solide blanc, F= 66°C.

L'(amino-2 méthyl-4 thiazolyl-5) acétate d'éthyle peut être préparé par la méthode décrite par H. YASUDA, J. Sci. Research Inst. (Tokyo) *51*, 32 (1957).

Les médicaments constitués par un nouveau dérivé de formule générale (I) à l'état pur, éventuellement sous forme de sel pharmaceutiquement acceptable, et les compositions pharmaceutiques qui le contiennent en association avec au moins un diluant ou adjuvant compatible et pharmaceutiquement acceptable, constituent un autre objet de la présente invention. Ces médicaments peuvent être employés par voie intranasale, orale, rectale, parentérale ou topique.

Comme compositions solides pour administration orale peuvent être utilisés des comprimés, des pilules, des poudres (de préférence contenues dans les capsules de gélatine) ou des granulés. Dans ces compositions, le produit actif selon l'invention est mélangé à un ou plusieurs diluants inertes tels que saccharose, lactose ou amidon. Ces compositions peuvent également comprendre des substances autres que les diluants, par exemple un lubrifiant tel que le stéarate de magnésium.

Comme compositions liquides pour administration orale peuvent être utilisés des émulsions pharmaceutiquement acceptables, des solutions, des suspensions, des sirops et des élixirs contenant des diluants inertes tels que l'huile de paraffine. Ces compositions peuvent également comprendre des substances autres que les diluants, par exemple des produits mouillants, édulcorants ou aromatisants.

Les compositions selon l'invention pour administration intranasale ou parentérale peuvent être des solutions stériles aqueuses ou non aqueuses, des suspensions ou des émulsions. Comme solvant ou véhicule, on puet employer le propylèneglycol, un polyéthyèneglycol, des huiles végétales en particulier l'huile d'olive, d'amande ou de coco et des esters organiques injectables, par exemple l'oléate d'éthyle. Ces compositions peuvent également contenir des adjuvants, en particulier des agents mouillants, émulsifiants ou dispersants (lécithine de soja). La stérilisation peut se faire de plusieurs façons, par exemple en incorporant à la composition des agents stérilisants, par irradiation, par chauffage ou par addition d'un agent conservateur. Elles peuvent être présentées sous forme de compositions solides stériles qui seront dissoutes au moment de l'emploi dans un milieu stérile injectable.

Les compositions pour administration rectale sont des suppositoires qui peuvent contenir, outre le produit actif, des excipients tels que le beurre de cacao ou un glycéride semi-synthétique approprié.

Les topiques se présentent notamment sous forme de pommades.

Les compositions selon l'invention sont particulièrement utiles en thérapeutique humaine pour leur action anti-virale. Elles sont notamment indiquées pour le traitement des affections virales des voies respiratoires.

En thérapeutique humaine, les doses dépendent de l'effet recherché et de la durée du traitement; elles sont généralement comprises entre 100 et 2000 mg par jour pour un adulte par voie orale. Elles peuvent atteindre 100 mg par jour au maximum par voie nasale (gouttes ou nébulisations).

D'une façon générale, le médecin déterminera la posologie qu'il estime la plus appropriée en fonction de l'âge, du poids et de tous autres facteurs propres au sujet à traiter.

Les exemples suivants, donnés à titre non limitatif, illustrent des compositions selon l'invention.

Exemple A

On prépare selon la technique habituelle des comprimés dosés à 100 mg de produit actif, ayant la composition suivante:

| | |
|---|---|
| — [pyridazinyl-3 imino]-3 tétrahydro-1,5,10,10a thiazolo [3,4-b] isoquinoléine-(S) | 0,100 g |
| — amidon | 0,110 g |
| — silice précipitée | 0,035 g |
| — stéarate de magnésium | 0,005 g |

Exemple B

On prépare selon la technique ci-après des comprimés dosés à 100 mg de produit actif, ayant la composition suivante:

| | |
|---|---|
| — [(carboxyméthyl-4 thiazolyl-2) imino]-3 tétrahydro-1,5,10,10a thiazolo [3,4-b] isoquinoléine-(S) | 0,100 g. |
| — amidon de blé | 0,137 g |
| — phosphate dicalcique | 0,040 g |
| — carboxyméthylamidon sodique | 0,015 g |
| — stéarate de magnésium | 0,008 g |

La substance active, le phosphate dicalcique et environ 90% de l'amidon sont mélangés puis tamisés (ouverture de maille: 0,5 mm). On ajoute un empois d'amidon à 10% préparé à partir de l'amidon mis en réserve, on granule la pâte obtenue, par passage à travers un tamis (ouverture de maille 0,8 mm) et on sèche les granulés en étuve vers 50°C. On ajoute alors le carboxyméthylamidon et le stéarate de magnésium et on presse en comprimés.

Exemple C

On prépare une solution huileuse à 1% pour administration par voie intranasale en dissolvant 1 g de (thiazolyl-2) imino-3 tétrahydro-1,5,10,10a thiazolo [3,4-b] isoquinoléine-(S) dans 100 cm3 d'huile d'olive à 40—50°C et en filtrant la solution obtenue sur filtre Millipore. Pour l'emploi, la solution est appliquée sur la muqueuse nasale à l'aide d'un compte-gouttes.

Exemple D

On prépare une solution aqueuse à 1% de produit actif, pour administration par voie intra-nasale, en dissolvant 1,07 g du sel de sodium de la [(carboxyméthyl-4 thiazolyl)-2 imino]-3 tétrahydro-1,5,10,10a thiazolo [3,4-b] isoquinoléine-(S) dans 100 cm3 d'eau distillée. Pour l'emploi, la solution est appliquée sur la muqueuse nasale à l'aide d'un compte gouttes.

**Revendications pour les Etats contractants: BE CH DE FR GB IT LI LU NL SE**

1. Un nouveau dérivé de la thiazolo [3,4-b] isoquinoléine caractérisé en ce qu'il répond à la formule générale:

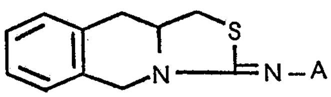

dans laquelle le symbole A représente un radical isoquinolyl-8, méthyl-3 isoquinolyl-8, hydroxyméthyl-3, isoquinolyl-5, carboxyméthyl-3 isoquinolyl-5, quinolyl-5, thiénopyridyle, benzimidazolyle, thiényle, thiazolyle, carboxyalcoyl-4 (ou -5) thiazolyl-2 dont la partie alcoyle est droite ou ramifiée et contient 1 à 4 atomes de carbone, thiadiazol-1,3,4 yl-2, pyrazolyle, imidazolyle, pyrimidinyle, pyridazinyle ou pyrazinyle, les hétérocycles monocyliques ci-dessus pouvant être substitués par un radical alcoyle droit ou ramifié contenant 1 à 4 atomes de carbone, sous forme (S) ou (R,S) ou leurs mélanges ainsi que, lorsque A est autre que thiényle, ses sels d'addition avec les acides et, lorsque A est carboxyméthyl-3 isoquinolyl-5 ou carboxyalcoyl-4 (ou -5) thiazolyl-2 éventuellement substitué par un radical alcoyle, ses sels métalliques ou ses sels d'addition avec les bases azotées.

2. Un procédé de préparation d'un produit selon la revendication 1, caractérisé en ce que l'on fait agir une amine de formule générale:

$$A—NH_2$$

dans laquelle A est défini selon la revendication 1, sur un sel de formule générale:

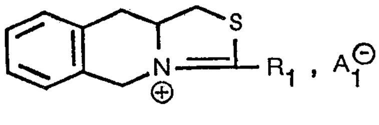

dans laquelle $R_1$ représente un atome de chlore et $A_1\ominus$ représente un ion chlorure, ou bien $R_1$ représente un radical alcoylthio contenant 1 à 4 atomes de carbone ou benzylthio et $A_1\ominus$ représente un

ion iodure, sulfate, tétrafluoroborate ou fluorosulfonate, puis transforme éventuellement le produit obtenu en sel d'addition avec un acide lorsque A est autre que thiényle, ou en un sel métallique ou sel d'addition avec une base azotée lorsque A contient un radical carboxy.

3. Un procédé de préparation d'un produit selon la revendication 1, pour lequel A est un radical isoquinolyl-8, méthyl-3 isoquinolyl-8, hydroxyméthyl-3 isoquinolyl-5, carboxyméthyl-3 isoquinolyl-5, thiénopyridyle, benzimidazolyl-6, quinolyl-5, pyrimidinyl-2 ou thiényl-2 (ces deux derniers étant éventuellement substitués par un radical alcoyle), caractérisé en ce que l'on cyclise en milieu acide une tétrahydro-1,2,3,4 isoquinoléine de formule générale:

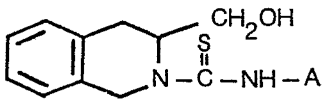

dans laquelle A est défini comme ci-dessus, puis transforme éventuellement le produit obtenu en un sel d'addition avec un acide lorsque A est autre que thiényle, ou en un sel métallique ou en un sel d'addition avec une base azotée lorsque A est carboxyméthyl-3 isoquinolyl-5.

4. Un procédé de préparation d'un produit selon la revendication 1, pour lequel A est hydroxyméthyl-3 isoquinolyl-5, caractérisé en ce que l'on réduit un ester de formule générale:

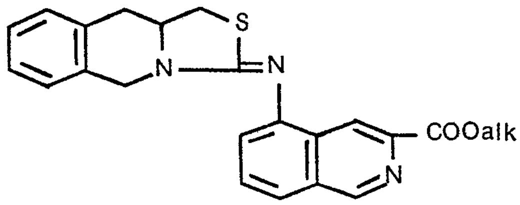

dans laquelle alk est un radical alcoyle contenant 1 à 4 atomes de carbone, puis transforme éventuellement le produit obtenu en un sel d'addition avec un acide.

5. Un procédé de préparation d'un produit selon la revendication 1, pour lequel A est carboxyméthyl-3 isoquinolyl-5 ou carboxyalcoyl-4 (ou -5) thiazolyl-2 éventuellement substitué par un radical alcoyle, caractérisé en ce que l'on transforme un ester de formule générale:

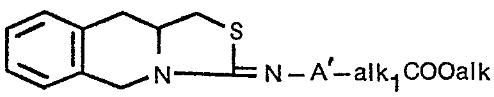

[dans laquelle alk est un radical alcoyle conenant 1 à 4 atomes de carbone, et soit —A' est un radical isoquinolyl-5 et $alk_1$ est un radical méthylène en position -3, soit —A' est un radical thiazolyl-2 éventuellement substitué par un radical alcoyle et $alk_1$ est un radical alcoylène droit ou ramifié, contenant 1 à 4 atomes de carbone, en position -4 (ou -5)] par toute méthode connue en soi pour obtenir un acide à partir d'un ester sans toucher au reste de la molécule, puis transforme éventuellement le produit obtenu en sel d'addition avec un acide, en sel métallique, ou en sel d'addition avec une base azotée.

6. Composition pharmaceutique caractérisé en ce qu'elle est constituée par un produit selon la revendication 1 en association avec un ou plusieurs diluants ou adjuvants compatibles et pharmaceutiquement acceptables.

**Revendication pour l'Etat contractant: AT**

Procédé pour la préparation d'un nouveau dérivé de la thiazolo [3,4-b] isoquinoléine de formule générale:

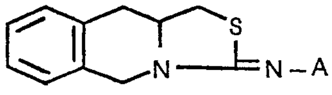

dans laquelle le symbole A représente un radical isoquinolyl-8, méthyl-3 isoquinolyl-8, hydroxyméthyl-3, isoquinolyl-5, carboxyméthyl-3 isoquinolyl-5, quinolyl-5, thiénopyridyle, benzimidazolyl, thiényle,

thiazolyle, carboxyalcoyl-4 (ou -5) thiazolyl-2 dont la partie alcoyle est droite ou ramifiée et contient 1 à 4 atomes de carbone, thiadiazol-1,3,4 yl-2, pyrazolyle, imidazolyle, pyrimidinyle, pyridazinyle ou pyrazinyle, les hétérocycles monocyliques ci-dessus pouvant être substitués par un radical alcoyle droit ou ramifié contenant 1 à 4 atomes de carbone, sous forme (S) ou (R,S) ou leurs mélanges ainsi que, lorsque A est autre que thiényle, ses sels d'addition avec les acides et, lorsque A est carboxyméthyl-3 isoquinolyl-5 ou carboxyalcoyl-4 (ou -5) thiazolyl-2 éventuellement substitué par un radical alcoyle, ses sels métalliques ou ses sels d'addition avec les bases azotées, caractérisé en ce que on fait agir une amine de formule générale:

$$A{-}NH_2$$

dans laquelle A possède la définition correspondante, sur un sel de formule générale:

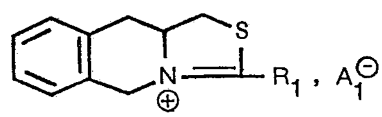

dans laquelle $R_1$ représente un atome de chlore et $A_1\ominus$ représente un ion chlorure, ou bien $R_1$ représente un radical alcoylthio contenant 1 à 4 atomes de carbone ou benzylthio et $A_1\ominus$ représente un ion iodure, sulfate, tétrafluoroborate ou fluorosulfonate, puis transforme éventuellement le produit obtenu en sel d'addition avec un acide lorsque A est autre que thiényle, ou en un sel métallique ou sel d'addition avec une base azotée lorsque A contient un radical carboxy ou,

pour la préparation d'un produit pour lequel A est un radical isoquinolyl-8, méthyl-3 isoquinolyl-8, hydroxyméthyl-3 isoquinolyl-5, carboxyméthyl-3 isoquinolyl-5, thiénopyridyle, benzimidazolyl-6, quinolyl-5, pyrimidinyl-2 ou thiényl-2 (ces deux derniers étant éventuellement substitués par un radical alcoyle), on cyclise en milieu acide un tétrahydro-1,2,3,4 isoquinoléine de formule générale:

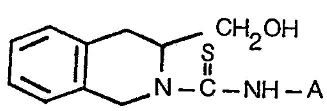

dans laquelle A possède la définition correspondante, puis transforme éventuellement le produit obtenu en un sel d'addition avec un acide lorsque A est autre que thiényle, ou en un sel métallique ou en un sel d'addition avec une base azotée lorsque A est carboxyméthyl-3 isoquinolyl-5 ou,

pour la préparation d'un produit pour lequel A est hydroxyméthyl-3 isoquinolyl-5, on réduit un ester de formule générale:

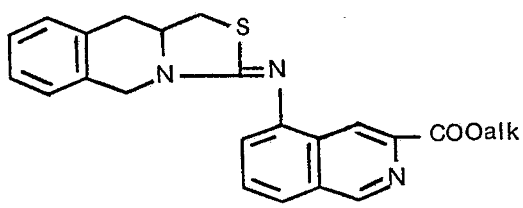

dans laquelle alk est un radical alcoyle contenant 1 à 4 atomes de carbone, puis transforme éventuellement le produit obtenu en un sel d'addition avec un acide ou,

pour la préparation d'un produit pour lequel A est carboxyméthyl-3 isoquinolyl-5 ou carboxyalcoyl-4 (ou -5) thiazolyl-2 éventuellement substitué par un radical alcoyle, on décompose un ester de formule générale:

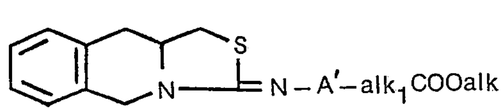

[dans laquelle alk est un radical alcoyle contenant 1 à 4 atomes de carbone, et soit —A' est un radical isoquinolyl-5 et $alk_1$ est un radical méthylène en position -3, soit —A' est un radical thiazolyl-2 éventuellement substitué par un radical alcoyle et $alk_1$ est un radical alcoylène droit ou ramifié,

contenant 1 à 4 atomes de carbone, en position -4 (ou -5)] pour obtenir l'acide correspondant, puis transforme éventuellement le produit en sel d'addition avec un acide, en sel métallique, ou en sel d'addition avec une base azotée.

**Claims for the Contracting States: BE CH DE FR GB IT LI LU NL SE**

1. A new thiazolo [3,4-b] isoquinoline derivative, characterised in that it corresponds to the general formula:

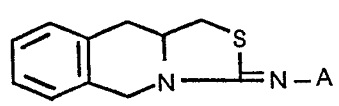

in which the symbol A represents an isoquinol-8-yl, 3-methylisoquinol-8-yl, 3-hydroxymethylisoquinol-5-yl, 3-carboxymethylisoquinol-5-yl, quinol-5-yl, thienopyridyl, benzimidazolyl, thienyl or thiazolyl radical, a 4-(or 5-)-carboxyalkylthiazol-2-yl radical, the alkyl part of which is linear or branched and contains 1 to 4 carbon atoms, or a 1,3,4-thiadiazol-2-yl, pyrazolyl, imidazolyl, pyrimidinyl, pyridazinyl or pyrazinyl radical, it being possible for the above monocyclic heterocyclic rings to be substituted by a linear or branched alkyl radical containing 1 to 4 carbon atoms, in the (S) or (R,S) form or in the form of mixtures thereof, and also, if A is other than thienyl, its addition salts with acids, and, if A is 3-carboxymethyisoquinol-5-yl or 4-(or 5-)carboxyalkylthiazol-2-yl optionally substituted by an alkyl radical, its metal salts or its addition salts with nitrogen-containing bases.

2. A process for the preparation of a product according to Claim 1, characterised in that an amine of the general formula:

$$A—NH_2$$

in which A is defined according to Claim 1, is reacted with a salt of the general formula:

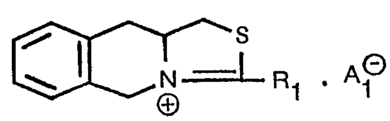

in which $R_1$ represents a chlorine atom and $A_1\ominus$ represents a chloride ion, or alternatively $R_1$ represents an alkylthio radical containing 1 to 4 carbon atoms, or a benzylthio radical, and $A_1\ominus$ represents an iodide, sulphate, tetrafluoroborate or fluorosulphonate ion, and the product obtained is then converted, if appropriate, to an addition salt with an acid if A is other than thienyl, or to a metal salt or an addition salt with a nitrogen-containing base if A contains a carboxyl radical.

3. A process for the preparation of a product according to Claim 1 in which A is an isoquinol-8-yl, 3-methyl-isoquinol-8-yl, 3-hydroxymethylisoquinol-5-yl, 3-carboxymethylisoquinol-5-yl, thienopyridyl, benzimidazol-6-yl, quinol-5-yl, pyrimidin-2-yl or thien-2-yl radical (these last two being optionally substituted by an alkyl radical), characterised in that a 1,2,3,4-tetrahydroisoquinoline of the general formula:

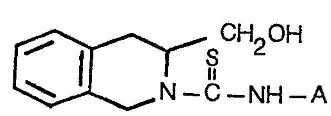

in which A is defined as above, is cyclised in an acid medium, and the product obtained is then converted, if appropriate, to an addition salt with an acid if A is other than thienyl, or to a metal salt or an addition salt with a nitrogen-containing base if A is 3-carboxymethylisoquinol-5-yl.

4. A process for the preparation of a product according to Claim 1 in which A is 3-hydroxymethylisoquinol-5-yl, characterised in that an ester of the general formula:

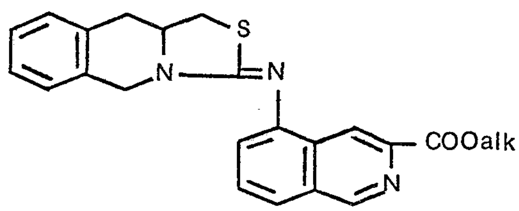

in which alk is an alkyl radical containing 1 to 4 carbon atoms, is reduced, and the product obtained is then converted, if appropriate, to an addition salt with an acid.

5. A process for the preparation of a product according to Claim 1 in which A is 3-carboxymethylisoquinol-5-yl or 4-(or 5-)carboxyalkylthiazol-2-yl optionally substituted by an alkyl radical, characterised in that an ester of the general formula:

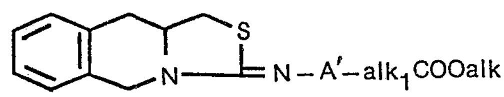

[in which alk is an alkyl radical containing 1 to 4 carbon atoms, and either —A′ is an isoquinol-5-yl radical and $alk_1$ is a methylene radical in the 3-position, or —A′ is a thiazol-2-yl radical optionally substituted by an alkyl radical and $alk_1$ is a linear or branched alkylene radical, containing 1 to 4 carbon atoms, in the 4-(or 5-) position] is converted by any method which is in itself known for obtaining an acid from an ester without affecting the rest of the molecule, and the product obtained is then converted, if appropriate, to an addition salt with an acid, a metal salt or an addition salt with a nitrogen-containing base.

6. Pharmaceutical composition, characterised in that it consists of a product according to Claim 1, in association with one or more compatible and pharmaceutically acceptable diluents or adjuvants.

**Claim for the Contracting State: AT**

Process for the preparation of a new thiazolo[3,4-b]isoquinoline derivative of the general formula:

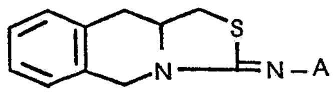

in which the symbol A represents an isoquinol-8-yl, 3-methylisoquinol-8-yl, 3-hydroxymethylisoquinol-5-yl, 3-carboxymethylisoquinol-5-yl, quinol-5-yl, thienopyridyl, benzimidazolyl, thienyl or thiazolyl radical, a 4-(or 5-)-carboxyalkylthiazol-2-yl radical, the alkyl part of which is linear or branched and contains 1 to 4 carbon atoms, or a 1,3,4-thiadiazol-2-yl, pyrazolyl, imidazolyl, pyrimidinyl, pyridazinyl or pyrazinyl radical, it being possible for the above monocyclic heterocyclic rings to be substituted by a linear or branched alkyl radical containing 1 to 4 carbon atoms, in the (S) or (R,S) form or mixtures thereof, and also, if A is other than thienyl, its addition salts with acids, and, if A is 3-carboxymethylisoquinol-5-yl or 4-(or 5)-carboxyalkylthiazol-2-yl optionally substituted by an alkyl radical, its metal salts or its addition salts with nitrogen-containing bases, characterised in that an amine of the general formula:

$$A—NH_2$$

in which A possesses the corresponding definition, is reacted with a salt of the general formula:

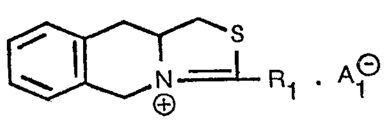

in which $R_1$ represents a chlorine atom and $A_1^{\ominus}$ represents a chloride ion, or alternatively $R_1$ represents an alkylthio radical containing 1 to 4 carbon atoms, or a benzylthio radical, and $A_1^{\ominus}$ represents an

iodide, sulphate, tetrafluoroborate or fluorosulphonate ion, and the product obtained is then converted, if appropriate, to an addition salt with an acid if A is other than thienyl, or to a metal salt or an addition salt with a nitrogen-containing base if A contains a carboxyl radical, or

for the preparation of a product in which A is an isoquinol-8-yl, 3-methylisoquinol-8-yl, 3-hydroxymethylisoquinol-5-yl, 3-carboxymethylisoquinol-5-yl, thienopyridyl, benzimidazol-6-yl, quinol-5-yl, pyrimidin-2-yl or thien-2-yl radical (these last two being optionally substituted by an alkyl radical), a 1,2,3,4-tetrahydroisoquinoline of the general formula:

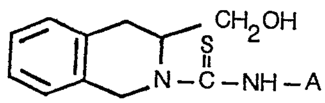

in which A possesses the corresponding definition, is cyclised in an acid medium, and the product obtained is then converted, if appropriate, to an addition salt with an acid if A is other than thienyl, or to a metal salt or an addition salt with a nitrogen-containing base if A is 3-carboxymethylisoquinol-5-yl, or

for the preparation of a product in which A is 3-hydroxymethylisoquinol-5-yl, an ester of the general formula:

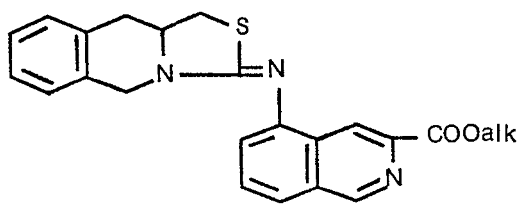

in which alk is an alkyl radical containing 1 to 4 carbon atoms, is reduced, and the product obtained is then converted, if appropriate, to an addition salt with an acid, or

for the preparation of a product in which A is 3-carboxymethylisoquinol-5-yl or 4(or 5)-carboxy-alkylthiazol-2-yl optionally substituted by an alkyl radical, an ester of the general formula:

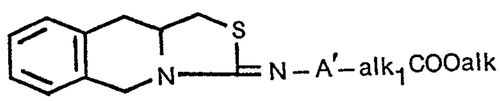

[in which alk is an alkyl radical containing 1 to 4 carbon atoms, and either —A′ is an isoquinol-5-yl radical and $alk_1$ is a methylene radical in the 3-position, or —A′ is a thiazol-2-yl radical optionally substituted by an alkyl radical and $alk_1$ is a linear or branched alkylene radical, containing 1 to 4 carbon atoms, in the 4(or 5)-position] is decomposed to give the corresponding acid, and the product is then converted, if appropriate, to an addition salt with an acid, a metal salt or an addition salt with a nitrogen-containing base.

**Patentansprüche für die Vertragsstaaten: BE CH DE FR GB IT LI LU NL SE**

1. Ein neues Derivat des Thiazolo[3,4-b]isochinolins, dadurch gekennzeichnet, daß es der allgemeinen Formel

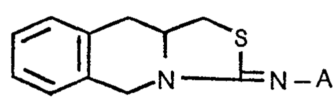

entspricht, worin das Symbol A bedeutet einen Rest Isochinolyl-(8), 3-Methyl-isochinolyl-(8), 3-Hydroxymethylisochinolyl-(5), 3-Carboxymethyl-isochinolyl-(5), Chinolyl-(5), Thienopyridyl, Benzimidazolyl, Thienyl, Thiazolyl, 4-(oder 5)Carboxyalkyl-thiazolyl-(2), dessen Alkylteil gerade oder verzweigt ist und 1 bis 4 Kohlenstoffatome enthält, 1,3,4-Thiadiazolyl-(2), Pyrazolyl, Imidazolyl, Pyrimidinyl, Pyridazinyl oder Pyrazinyl, wobei die obigen monocyclischen Heterocyclen durch einen geraden oder verzweigten Alkylrest mit 1 bis 4 Kohlenstoffatomen substituiert sein können, in der (S)- oder (R,S)-Form oder ihre Gemische, sowie, wenn A anders als Thienyl ist, sein Additionssalze mit Säuren, und wenn A

3-Carboxymethyl-isochinolyl-(5) oder 4-(oder 5)Carboxyalkylthiazolyl-(2), gegebenenfalls substituiert durch einen Alkylrest, ist, seine Metallsalze oder seine Additionssalze mit Stickstoffbasen.

2. Verfahren zur Herstellung eines Produkts gemäß Anspruch 1, dadurch gekennzeichnet, daß man ein Amin der allgemeinen Formel

$$A—NH_2$$

worin A wie in Anspruch 1 definiert ist, auf ein Salz der allgemeinen Formel

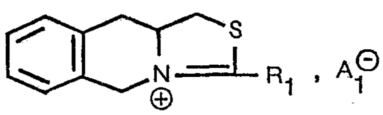

einwirken läßt, worin $R_1$ ein Chloratom bedeutet und $A_1\ominus$ ein Chloridion darstellt oder $R_1$ einen Alkylthiorest mit 1 bis 4 Kohlenstoffatomen oder Benzylthio bedeutet und $A_1\ominus$ ein Jodid-, Sulfat-, Tetrafluorborat- oder Fluorsulfonation bedeutet, und daß man dann gegebenenfalls das erhaltene Produkt in ein Additionssalze mit einer Säure überführt, falls A anders als Thienyl ist, oder in ein Metallsalz oder Additionssalz mit einer Stickstoffbase, falls A einen Carboxyrest enthält.

3. Verfahren zur Herstellung eines Produkts gemäß Anspruch 1, wobei A ein Rest Isochinolyl-(8), 3-Methyl-isochinolyl-(8), 3-Hydroxymethyl-isochinolyl-(5), 3-Carboxymethyl-isochinolyl-(5), Thienopyridyl, Benzimidazolyl-(6), Chinolyl-(5), Pyrimidinyl-(2) oder Thienyl-(2) ist (wobei die beiden letzteren gegebenenfalls durch einen Alkylrest substituiert sind), dadurch gekennzeichnet, daß man in saurem Milieu ein 1,2,3,4-Tetrahydro-isochinolin der allgemeinen Formel

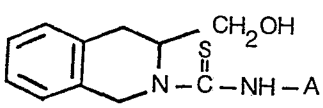

worin A wie oben definiert ist, cyclisiert, und daß man dann gegebenenfalls das erhaltene Produkt in ein Additionssalz mit einer Säure überführt, falls A etwas anderes als Thienyl ist, oder in ein Metallsalz oder ein Additionssalz mit einer Stickstoffbase, wenn A 3-Carboxymethyl-isochinolyl-(5) ist.

4. Verfahren zur Herstellung eines Produkts gemäß Anspruch 1, worin A 3-Hydroxymethyl-isochinolyl-(5) ist, dadurch gekennzeichnet, daß man einen Ester der allgemeinen Formel

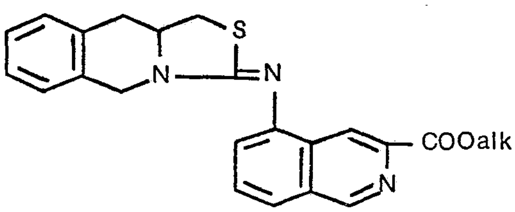

worin alk ein Alkylrest mit 1 bis 4 Kohlenstoffatomen ist, reduziert, und daß man dann gegebenenfalls das erhaltene Produkt in ein Additionssalz mit einer Säure überführt.

5. Verfahren zur Herstellung eines Produkts gemäß Anspruch 1, wobei A 3-Carboxymethyl-isochinolyl-(5) oder 4-(oder 5)Carboxyalkyl-thiazolyl-(2), gegebenenfalls substituiert durch einen Alkylrest, ist, dadurch gekennzeichnet, daß man einen Ester der allgemeinen Formel

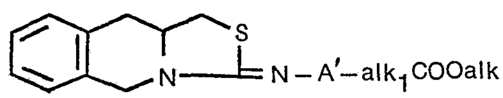

[worin alk ein Alkylrest mit 1 bis 4 Kohlenstoffatomen ist und entweder —A' ein Isochinolyl-(5)-Rest ist und $alk_1$ ein Methylenrest in 3-Stellung ist, oder —A' ist ein Thiazolyl-(2)-Rest, gegebenenfalls substituiert durch einen Alkylrest, und $alk_1$ ist ein gerader oder verzweiger Alkylenrest mit 1 bis 4 Kohlenstoffatomen in 4-(oder 5)Stellung] nach jeder an sich bekannten Methode zur Erzielung einer Säure, ausgehend von einem Ester, umwandelt, ohne daß der Rest des Moleküls berührt wird, und daß man dann gegebenenfalls das erhaltene Produkt in ein Additionssalz mit einer Säure, ein Metallsalz oder ein Additionssalz mit einer Stickstoffbase überführt.

6. Pharmazeutische Zusammensetzung, dadurch gekennzeichnet, daß sie aus einem Produkt gemäß Anspruch 1 zusammen mit einem oder mehreren verträglichen und pharmazeutisch annehmbaren Verdünnungsmitteln oder Hilfsmitteln besteht.

**Patentanspruch für den Vertragsstaat: AT**

Verfahren zur Herstellung eines neuen Thiazolo [3,4-b]-isochinolinderivats der allgemeinen Formel

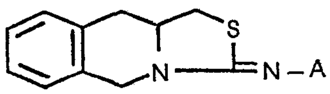

in welcher das Symbol A einen 8-Isochinolyl-, 3-Methyl-8-isochinolyl-, 3-Hydroxymethyl-5-isochinolyl-, 3-Carboxymethyl-5-isochinolyl-, 5-Chinolyl, Thienopyridyl-, Benzimidazolyl-, Thienyl-, Thiazolyl-, 4-(oder 5-)Carboxyalkyl-2-thiazolylrest, dessen Alkylteil gerade oder verzweigt ist und 1 bis 4 Kohlenstoffatome enthält, einen 1,3,4-Thiadiazol-2-yl-, Pyrazolyl-, Imidazolyl-, Pyrimidinyl-, Pyridazinyl- oder Pyrazinylrest darstellt, wobei die obigen monocylischen Heterocyclen durch eine geradkettige oder verzweigte Alkylgruppe mit 1 bis 4 Kohlenstoffatomen substituiert sein können, in der (S)- oder (R,S)-Form oder deren Mischungen sowie, wenn A von Thienyl verschieden ist, seiner Säureadditionssalze und, wenn A 3-Carboxymethyl-5-isochinolyl oder 4-(oder 5-)Carboxyalkyl-2-thiazolyl, das gegebenenfalls durch eine Alkylgruppe substituiert ist, ist, seiner Metallsalze oder seiner Additionssalze mit Stickstoffbasen, dadurch gekennzeichnet, daß man ein Amin der allgemeinen Formel

$$A—NH_2 ,$$

worin A die oben angegebene Bedeutung hat, auf ein Salz der allgemeinen Formel

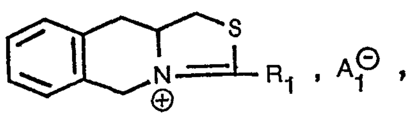

worin $R_1$ ein Chloratom darstellt und $A_1\ominus$ ein Chloridion bedeutet oder aber $R_1$ einen Alkylthiorest mit 1 bis 4 Kohlenstoffatomen oder Benzylthiorest darstellt und $A_1\ominus$ für ein Jodid-, Sulfat-, Tetrafluorborat- oder Fluorsulfonation steht, einwirken läßt, und das erhaltene Produkt dann gegebenenfalls in ein Säureadditionssalz überführt, wenn A von Thienyl verschieden ist, oder in ein Metallsalz oder ein Salz mit einer Stockstoffbase überführt, wenn A einen Carboxyrest enthält oder daß man zur Herstellung einer Verbindung, in welcher A ein 8-Isochinolyl-, 3-Methyl-8-isochinolyl-, 3-Hydroxymethyl-5-isochinolyl-, 3-Carboxymethyl-5-isochinolyl-, Thienopyridyl-, 6-Benzimidazolyl-, 5-Chinolyl-, 2-Pyrimidinyl- oder 2-Thienylrest ist (welche beiden letzten gegebenenfalls durch eine Alkylgruppe substituiert sind), in saurem Milieu ein 1,2,3,4-Tetrahydro-isochinolin der allgemeinen Formel

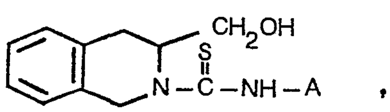

worin A die obige Bedeutung hat, cyclisiert, und dann das erhaltene Produkt gegebenenfalls in ein Säureadditionssalz wenn A von Thineyl verschieden ist, oder in ein Metallsalz oder ein Additionssalz mit einer Stickstoffbase überführt, wenn A 3-Carboxymethyl-5-isochinolyl ist oder daß man zur Herstellung einer Verbindung, in welcher A 3-Hydroxymethyl-5-isochinolyl ist, einen Ester der allgemeinen Formel

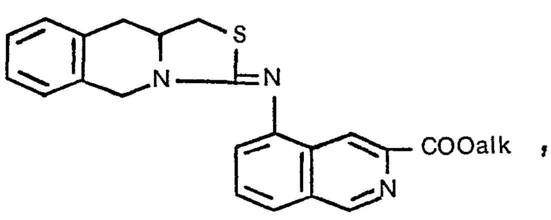

in welcher alk ein Alkylgruppe mit 1 bis 4 Kohlenstoffatomen ist, reduziert, und das erhaltene Produkt gegebenenfalls in ein Säureadditionssalz überführt oder daß man zur Herstellung einer Verbindung, in welcher A 3-Carboxymethyl-5-isochinolyl oder gegebenenfalls durch eine Alkylgruppe substituiertes 4- (oder 5-) Carboxyalkyl-2-thiazolyl ist, einen Ester der allgemeinen Formel

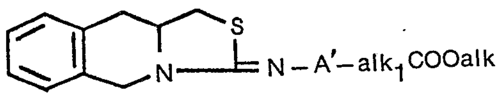

[in welcher alk eine Alkylgruppe mit 1 bis 4 Kohlenstoffatomen ist und entweder —A' eine 5-Isochinolylgruppe und $alk_1$ eine Methylengruppe in 3-Stellung oder —A' ein gegebenenfalls durch eine Alkylgruppe substituierter 2-Thiazolylrest und $alk_1$ eine geradkettige oder verzweigte Alkylengruppe mit 1 bis 4 Kohlenstoffatomen in 4-(oder 5-)-Stellung ist] zur Herstellung der entsprechenden Säure umwandelt, und dann das erhaltene Produkt gegebenenfalls in ein Säureadditionssalz, ein Metallsalz oder ein Additionssalz mit einer Stickstoffbase überführt.